# EUROPEAN PATENT SPECIFICATION

(11) **EP 4 339 205 B1**
(45) Date of publication and mention of the grant of the patent: **30.09.2026**
(21) Application number: 22810651.4
(22) Date of filing: 27.05.2022
(51) Int. Cl.: C07K 16/14, G01N 33/569

(54) **INDICATOR MOLECULE FOR TOXIGENICITY OF AFLATOXIGENIC FUNGI AND USE THEREOF**
INDIKATORMOLEKÜL FÜR DIE TOXIGENITÄT VON AFLATOXINEN PILZEN UND VERWENDUNG DAVON
MOLÉCULE INDICATRICE POUR LA TOXIGÉNICITÉ DE CHAMPIGNONS AFLATOXIGÈNES ET SON UTILISATION

(30) Priority: 28.05.2021 CN 202110591612
(43) Date of publication of application: 20.03.2024
(73) Proprietor: Oil Crops Research Institute, Chinese Academy of Agricultural Sciences, Wuhan, Hubei 430062 (CN)
(72) Inventor: LI, Peiwu, Wuhan, Hubei 430062 (CN); ZHANG, Qi, Wuhan, Hubei 430062 (CN); BAI, Yizhen, Wuhan, Hubei 430062 (CN)
(74) Representative: Lorenz Seidler Gossel Part. mbB
(86) International application number: PCT/CN2022/095521
(87) International publication number: WO 2022/247927

(56) References cited:
- CN-A- 102 746 403
- CN-A- 105 418 760
- CN-A- 113 480 624
- CN-A- 113 484 512
- CN-A- 113 607 949
- CN-A- 113 607 959
- CN-A- 113 621 676
- CN-A- 113 640 510
- CN-A- 114 097 459
- CN-B- 109 557 314
- TING HE, YANRU WANG, PEIWU LI , QI ZHANG , JIAWEN LEI , ZHAOWEI ZHANG† , XIAOXIA DING , HAIYAN ZHOU , WEN ZHANG: "Nanobody-based enzyme immunoassay for aflatoxin in agro-products with high tolerance to cosolvent methanol.", ANALYTICAL CHEMISTRY, vol. 86, no. 17, 2 September 2014 (2014-09-02), USA , pages 8873 - 8880, XP002772073, ISSN: 1520-6882, DOI: 10.1021/ac502390c
- YANRU WANG, PEIWU LI, ZUZANA MAJKOVA, CANDACE R. S. BEVER, HEE JOO KIM, QI ZHANG, JULIE E. DECHANT, SHIRLEY J. GEE, BRUCE D. HAMMO: "Isolation of alpaca anti-idiotypic heavy-chain single-domain antibody for the aflatoxin immunoassay.", ANALYTICAL CHEMISTRY, vol. 85, no. 17, 3 September 2013 (2013-09-03), USA , pages 8298 - 8303, XP002772072, ISSN: 1520-6882, DOI: 10.1021/ac4015885
- ZHAO FENGCHUN, TIAN YUAN, SHEN QIANG, LIU RUXIA, SHI RUIRUI, WANG HUIMIN, YANG ZHENGYOU: "A novel nanobody and mimotope based immunoassay for rapid analysis of aflatoxin B1", TALANTA, vol. 195, 1 April 2019 (2019-04-01), NL , pages 55 - 61, XP093007706, ISSN: 0039-9140, DOI: 10.1016/j.talanta.2018.11.013
- ZHANG XING: "Study on the Distribution,Toxigenicity and Infection of Aspergillus Flavus in Typical Peanut Producing Areas in China", CHINESE MASTER'S THESES FULL-TEXT DATABASE, 1 June 2019 (2019-06-01), pages 1 - 89, XP093007713
- TANG ZAO-XIU, JI RONG-CHANG, LI GUANG-XING, KANG YU-MEI,CHONG ZANG: "Toxigenicity and Pathogenicity of Groundnut A Flavus Strain", FUJIAN JOURNAL OF AGRICULTURAL SCIENCES, vol. 15, no. 2, 30 June 2000 (2000-06-30), pages 19 - 22, XP093007719, ISSN: 1008-0384, DOI: 10.19303/j.issn.1008-0384.2000.02.004

## Description

### TECHNICAL FIELD

The present disclosure relates to an indicator molecule for toxigenicity of aflatoxigenic fungi and use thereof.

### BACKGROUND

Aflatoxins are highly toxic and very harmful, and are pollutants that pollute the most types of food, and have generally shown a tendency to aggravate pollution in recent years, which seriously threatens food safety and people's health. Aflatoxins are the most toxic mycotoxins in nature, among which aflatoxin B 1, a class I carcinogen identified by the International Agency for Research on Cancer (IARC), has caused multiple poisoning incidents in human and animal populations, and has become one of the main reasons for the high incidence of liver cancer cases. According to the statistics of retrieval data from Web of Science in recent 5 years: aflatoxins pollute more than 110 types of foods and raw materials, ranking first among pollutants. However, to date, there is still no molecular warning research example prior to pollution with microbial toxins such as aflatoxins in China and abroad, and it is difficult to meet the urgent needs of early warning.

The existing aflatoxin early warning methods are mainly established based on an aflatoxin detection technology for toxin pollution level evaluation or evaluation of a pollution level after production and a consumption risk, once aflatoxin is detected, pollution often occurs, and it is difficult to meet the urgent needs of early warning and guided control. The European Union Rapid Alert System for Food and Feed (RASFF) performs rapid warning on food and feed imported into the European Union by various countries by using limit criteria and testing the content of aflatoxin in food and feed. Based on the aflatoxin detection technology and pollution monitoring data, a research institute in the United States has established early warning models such as multiple logistic regression analysis and stacked Gaussian treatment, mainly for assessing mycotoxin pollution levels and consumption risks for agricultural products such as corn after production.

According to the research progress in China and abroad in recent twenty years, a root cause is that an aflatoxin early warning molecule is unknown at present. For this bottleneck problem, after more than ten years of research on key problems, the inventor team has constructed an aflatoxin-producing strain library, a strain toxigenicity database, and a toxigenic strain protein antibody library in China, established a method for discovering an antibody library of indicator molecules for toxigenicity of Aspergillus flavus strains, and successfully invented an indicator molecule for toxigenicity of aflatoxigenic fungi which is used to identify the aflatoxin-producing ability of strains, and discover whether there are aflatoxin-producing strains with high toxigenicity in farmland, agricultural products and feed,, providing scientific basis for timely discovery of aflatoxin pollution risks and early prevention and early control.

CN 109 557 314 B discloses a method for determining toxigenicity of aflatoxigenic fungi.

### SUMMARY

In view of the shortcomings in the prior art, the present disclosure provides an indicator molecule AFT-YJFZ01 for toxigenicity of aflatoxigenic fungi, and a method for identifying the aflatoxin-producing ability of an Aspergillus strain in a system. The indicator molecule AFT-YJFZ01 for toxigenicity of aflatoxigenic fungi is used to identify the aflatoxin-producing ability of an Aspergillus strain, and identify whether there is an aflatoxin-producing strain with high toxigenicity in agricultural fields, agricultural products and feed, which is easy to popularize and apply.

To solve the above technical problems, the technical solutions adopted by the present disclosure are as follows:

provided is an indicator molecule AFT-YJFZ01 for toxigenicity of aflatoxigenic fungi, having the amino acid sequence shown in SEQ ID NO 1.

Provided is a method for identifying toxigenicity of aflatoxigenic fungi in a system , including detecting the content of an indicator molecule AFT-YJFZ01 for toxigenicity of aflatoxigenic fungi, and identifying the toxigenicity of aflatoxigenic fungi in a system on the basis of the content of the indicator molecule AFT-YJFZ01 for toxigenicity of aflatoxigenic fungi, having the amino acid sequence shown in SEQ ID NO. 1.

In particular, an antibody corresponding to the protein is prepared by adopting the amino acid sequence of the indicator molecule AFT-YJFZ01 for toxigenicity of aflatoxigenic fungi or a partial sequence therein by a conventional antibody preparation process, and quantitative detection of the indicator molecule protein is achieved. Quantitative detection having a one-to-one correspondence with the protein can also be achieved by other detection techniques, thereby achieving the above purpose. The partial sequence refers to a portion of a complete sequence having a one-to-one correspondence with the indicator molecule protein.

Provided is method use of identifying the aflatoxin-producing ability of an Aspergillus aflatoxin-producing strain, wherein the higher the content of indicator molecule AFT-YJFZ01 for toxigenicity of aflatoxigenic fungi in a strain to be identified, the stronger the aflatoxin-producing ability, that is, toxigenicity, of the strain to be identified; and
a specific application method includes:
   (1) providing a nanobody or monoclonal antibody of the indicator molecule AFT-YJFZ01 for toxigenicity of aflatoxigenic fungi;
   (2) providing a polyclonal antibody of the indicator molecule AFT-YJFZ01 for toxigenicity of aflatoxigenic fungi;
   (3) preparation of a to-be-tested solution of a strain to be identified: culturing the strain to be identified, and diluting to obtain the to-be-tested solution of the strain to be identified;
specifically, culturing the strain to be identified in a conventional Czapek medium or other media suitable for growth of strains at an ambient temperature of 15-35°C for not less than 12 hours, taking a mixture of the medium and a culture to be well homogenized, and performing diluting by 1-10 times with sterile water to obtain the to-be-tested solution of the strain to be identified; and
(4) determination of the aflatoxin-producing ability of the strain to be identified:
identifying the aflatoxin-producing ability of the Aspergillus aflatoxin-producing strain by using an indirect non-competitive double antibody sandwich method, including the steps of: coating an ELISA plate with the nanobody or monoclonal antibody of AFT-YJFZ01, and washing the plate; performing blocking by adding a blocking solution, and washing the plate; adding the to-be-tested solution, carrying out a reaction, and washing the plate; adding the polyclonal antibody of AFT-YJFZ01, carrying out a reaction, and washing the plate; adding a horseradish peroxidase labeled antibody that binds to the polyclonal antibody of the indicator molecule AFT-YJFZ01 for toxigenicity of aflatoxigenic fungi, carrying out a reaction, and washing the plate; adding a chromogen solution, and carrying out a reaction; and adding a stop solution, performing reading by using a microplate reader, and performing calculation to obtain the content of the indicator molecule AFT-YJFZ01 for toxigenicity of aflatoxigenic fungi, wherein the higher the content of the indicator molecule AFT-YJFZ01 for toxigenicity of aflatoxigenic fungi in the to-be-tested solution of the strain to be identified, the stronger the aflatoxin-producing ability of the aflatoxin-producing strain.

The specific steps include:
Step a, preparing a coating solution of 0.2-8.0 µg/mL from the nanobody or monoclonal antibody of the AFT-YJFZ01 by using an ELISA coating buffer, adding the coating solution into an ELISA plate (200 µL/well), removing the coating solution from the ELISA plate after standing overnight at 4°C or at 37°C for not less than 2h, and washing the ELISA plate with a conventional washing solution for ELISA; and then performing blocking at room temperature or 37°C for not less than 1h by using skimmed milk powder having a concentration of not less than 1% as a blocking solution (300 µL/well), discarding the blocking solution, and washing the ELISA plate with the conventional washing solution for ELISA;
Step b, then appropriately diluting the to-be-tested solution with a conventional phosphate buffer with a pH close to be neutral, adding the diluted to-be-tested solution to wells of the ELISA plate (200 µL/well), performing blocking at room temperature or 37°C for not less than 1h, discarding a liquid, and washing the ELISA plate with the conventional washing solution for ELISA;
Step c, then appropriately diluting the polyclonal antibody of the AFT-YJFZ01 with a conventional phosphate buffer with a pH close to be neutral, adding the diluted polyclonal antibody into wells of the ELISA plate (200 µL/well), performing blocking at room temperature or 37°C for not less than 1h, discarding a liquid, and washing the ELISA plate with the conventional washing solution for ELISA;
Step d, then diluting a commercial horseradish peroxidase labeled antibody with a conventional phosphate buffer with a pH close to be neutral as needed, adding the diluted commercial horseradish peroxidase labeled antibody into wells of the ELISA plate (200 µL/well), performing blocking at room temperature or 37°C for not less than 1h, discarding a liquid, and washing the ELISA plate with the conventional washing solution for ELISA; and
Step e, then sequentially adding a conventional chromogen solution for ELISA and a stop solution, and finally reading by using a microplate reader, and calculating the content result of AFT-YJFZ01.

According to the above solution, solutions of the indicator molecule AFT-YJFZ01 with a series of concentration gradients are used to make a standard curve, and the content of AFT-YJFZ01 is obtained by using the standard curve based on the results of the microplate reader. That is, the to-be-tested solution of the strain to be identified is replaced with the solutions of the indicator molecule AFT-YJFZ01 with a series of concentration gradients in the step b to make the standard curve.

Provided is an application of identifying whether there is an aflatoxin-producing strain with high toxigenicity in a sample, wherein a specific application method includes the following steps:
(1) providing a nanobody or monoclonal antibody of the indicator molecule AFT-YJFZ01 for toxigenicity of aflatoxigenic fungi;
(2) providing a polyclonal antibody of the indicator molecule AFT-YJFZ01 for toxigenicity of aflatoxigenic fungi;
(3) preparation of a to-be-tested solution of a sample to be identified: culturing the sample to be identified, and diluting to obtain the to-be-tested solution of the sample to be identified;
   specifically: weighing the sample to be identified, transferring the weighed sample to be identified to an appropriate amount of sterile water, performing shaking at room temperature until the obtained solution is uniform to prepare a uniform dispersion of the sample to be tested, taking 10-1000 µL of the uniform dispersion of the sample to be added into 6-600 mL of a conventional Czapek medium or other media suitable for growth of Aspergillus flavus producing toxins, performing shaking culture at 15-35°C at 200±50 rpm after 6-24 h, and taking a sample to form the to-be-tested solution of the sample to be identified.
(4) identifying whether there is an aflatoxin-producing strain with high toxigenicity in a sample: including the steps of:
   coating an ELISA plate with the nanobody or monoclonal antibody of AFT-YJFZ01, and washing the plate; performing blocking by adding a blocking solution, and washing the plate; adding the to-be-tested solution of the sample to be identified, carrying out a reaction, and washing the plate; adding the polyclonal antibody of AFT-YJFZ01, carrying out a reaction, and washing the plate; adding a horseradish peroxidase labeled antibody that binds to the polyclonal antibody of the indicator molecule AFT-YJFZ01 for toxigenicity of aflatoxigenic fungi, carrying out a reaction, and washing the plate; adding a chromogen solution, and carrying out a reaction; and adding a stop solution, performing reading by using a microplate reader, and calculating the content of AFT-YJFZ01;

The specific steps include:
Step a, preparing a coating solution of 0.2-8.0 µg/mL from the nanobody or monoclonal antibody of the AFT-YJFZ01 by using an ELISA coating buffer, adding the coating solution into an ELISA plate (200 µL/well), removing the coating solution from the ELISA plate after standing overnight at 4°C or at 37°C for not less than 2h, and washing the ELISA plate with a conventional washing solution for ELISA; and then performing blocking at room temperature or 37°C for not less than 1h by using skimmed milk powder having a concentration of not less than 1% as a blocking solution (300 µL/well), discarding the blocking solution, and washing the ELISA plate with the conventional washing solution for ELISA;
Step b, then appropriately diluting the to-be-tested solution of the sample to be identified with a conventional phosphate buffer with a pH close to be neutral, adding the diluted to-be-tested solution to wells of the ELISA plate (200 µL/well), performing blocking at room temperature or 37°C for not less than 1h, discarding a liquid, and washing the ELISA plate with the conventional washing solution for ELISA;
Step c, then appropriately diluting the polyclonal antibody of the AFT-YJFZ01 with a conventional phosphate buffer with a pH close to be neutral, adding the diluted polyclonal antibody into wells of the ELISA plate (200 µL/well), performing blocking at room temperature or 37°C for not less than 1h, discarding a liquid, and washing the ELISA plate with the conventional washing solution for ELISA;
Step d, then diluting a commercial horseradish peroxidase labeled antibody with a conventional phosphate buffer with a pH close to be neutral as needed, adding the diluted commercial horseradish peroxidase labeled antibody into wells of the ELISA plate (200 µL/well), performing blocking at room temperature or 37°C for not less than 1h, discarding a liquid, and washing the ELISA plate with the conventional washing solution for ELISA; and
Step e, then sequentially adding a conventional chromogen solution for ELISA and a stop solution, and finally reading by using a microplate reader, and calculating the content result of AFT-YJFZ01.

(5) Evaluation of identification results:
the higher the content of the indicator molecule AFT-YJFZ01 for toxigenicity of aflatoxigenic fungi in the sample to be identified, the stronger the aflatoxin-producing ability, that is, the toxigenicity, of the sample to be identified, obtaining the content of the indicator molecule AFT-YJFZ01 for toxigenicity of aflatoxigenic fungi in the to-be-tested solution of the sample per unit volume based on the result of calculation by the microplate reader, and determining whether an aflatoxin-producing strain with high toxigenicity is contained in the sample to be identified.

According to the above solution, the sample to be identified is soil, an agricultural product, a traditional Chinese medicine, a feed, or the like.

According to the above solution, solutions of the indicator molecule AFT-YJFZ01 with a series of concentration gradients are used to make a standard curve, and the content of AFT-YJFZ01 is obtained by using the standard curve based on the results of the microplate reader. That is, the to-be-tested solution of the sample to be identified is replaced with the solutions of the indicator molecule AFT-YJFZ01 with a series of concentration gradients in the step b to make the standard curve. According to the above solution, an animal source of the polyclonal antibody of the indicator molecule AFT-YJFZ01 for toxigenicity of aflatoxigenic fungi is different from an animal source of the nanobody or monoclonal antibody of the indicator molecule AFT-YJFZ01 for toxigenicity of aflatoxigenic fungi, in particular:
the nanobody or monoclonal antibody of the indicator molecule AFT-YJFZ01 for toxigenicity of aflatoxigenic fungi can be developed by immunizing alpacas or Balb/c mice in a conventional manner by using AFT-YJFZ01 as an immunizing antigen, and then using a known conventional nanobody or murine monoclonal antibody preparation technical solution;
the polyclonal antibody of the indicator molecule AFT-YJFZ01 for toxigenicity of aflatoxigenic fungi can be obtained by using the following method: immunizing test rabbits such as New Zealand white rabbits in a conventional manner by using AFT-YJFZ01 as an immunizing antigen, and developing a rabbit-derived polyclonal antibody of the indicator molecule AFT-YJFZ01 for toxigenicity of aflatoxigenic fungi by using a known conventional polyclonal antibody preparation technical solution; and
the horseradish peroxidase labeled antibody that binds to the polyclonal antibody of the indicator molecule AFT-YJFZ01 for toxigenicity of aflatoxigenic fungi is a horseradish peroxidase labeled goat anti-rabbit antibody, and can be obtained by directly purchasing a commercial product.

The toxigenicity of an Aspergillus flavus strain is a measure of the aflatoxin-producing ability of the strain, the stronger the toxigenicity of the strain, the more aflatoxin can be produced by the strain at the same time and under the same culture conditions. The present disclosure provides the indicator molecule AFT-YJFZ01 for toxigenicity of aflatoxigenic fungi, and further provides applications of identifying the aflatoxin-producing ability of the Aspergillus aflatoxin-producing strain, and identifying whether there is an aflatoxin-producing strain with high toxigenicity in agricultural fields, agricultural products and feed by using the feature that the content of the indicator molecule AFT-YJFZ01 for toxigenicity of aflatoxigenic fungi is positively correlated with the toxigenicity of the strain, which is practical and easy to popularize, provides a key grip and scientific basis for timely discovery of aflatoxin pollution risks and early prevention and early control, and has important significance for promoting high-quality development of the agricultural industry and ensuring the food safety.

The beneficial effects of the present disclosure are as follows:
1. the indicator molecule AFT-YJFZ01 for toxigenicity of aflatoxigenic fungi can be used to identify the aflatoxin-producing ability of the Aspergillus aflatoxin-producing strain;
2. the indicator molecule AFT-YJFZ01 for toxigenicity of aflatoxigenic fungi can be used to identify whether there is an aflatoxin-producing strain with high toxigenicity in agricultural fields, agricultural products and feed;
3. the method is easy to operate, high in practicality, and easy to popularize and apply; and
4. the indicator molecule AFT-YJFZ01 for toxigenicity of aflatoxigenic fungi and the applications thereof have important significance for promoting high-quality development of the agricultural industry and ensuring the food safety.

### DETAILED DESCRIPTION

### Example 1 Preparation of an indicator molecule AFT-YJFZ01 for toxigenicity of aflatoxigenic fungi

A Czapek medium was prepared according to the following formula: 3% (w/v) sucrose, 0.3% (w/v) NaNO₃, 0.1% (w/v) K₂HPO₄, 0.05% (w/v) MgSO₄·7H₂O, 0.05% (w/v) KCl, and 0.001% (w/v) FeSO₄, with a pH of 6.5. Ten published toxigenic strains, including HLJ-1, HeNZY-2, HuBha-24, JXZS-29-2, LNct-6, GXfc-34, GDZJ-122-2, JSnt-1, HuNdx-7, and HBHA-8-17 were randomly selected from Page 33 of the published literature "Study on Distribution,Toxigenicity and Infection of Aspergillus flavus in Typical Peanut Producing Areas of China"--Master's Thesis of Chinese Academy of Agricultural Sciences, of which the author is Zhang Xing, were separately inoculated into the above-mentioned Czapek medium to be cultured at 28°C at 200 rpm/min for 5 days, were homogenized well in a conventional manner to disrupt cells, and were purified by using a conventional protein purification system, protein electrophoresis, immunoaffinity, etc. to obtain the indicator molecule AFT-YJFZ01 for toxigenicity of aflatoxigenic fungi. The test results show that AFT-YJFZ01 can be prepared in cultures of the above toxigenic strains. Under the same culture conditions, HBHA-8-17 produces the greatest amount of AFT-YJFZ01 and HLJ-1 produces the least amount of AFT-YJFZ01.

The indicator molecule AFT-YJFZ01 for toxigenicity of aflatoxigenic fungi was initially obtained by a discovery method:
the discovery method for the indicator molecule AFT-YJFZ01 for toxigenicity of Aspergillus flavus strain was as follows:
(1) an *Aspergillus flavus* strain with high toxigenicity was taken to be cultured to obtain a strain culture and an extracellular secreted protein mixture; cells of the strain culture were then disrupted to obtain an intracellular protein mixture; and the above extracellular secreted protein mixture was mixed with the intracellular protein mixture, and the obtained mixture was coupled by adding carbodiimide to obtain an Aspergillus flavus antigen;
(2) a test animal was immunized with the above Aspergillus flavus antigen to obtain a nanobody library or a monoclonal antibody library;
(3) a mixed solution of proteins of *Aspergillus flavus* strains with different toxigenicity was obtained, and the proteins of the Aspergillus flavus strains with different toxigenicity were detected by using antibodies in the antibody library obtained in the step (2) to obtain serial detection signals; and
(4) a nanobody or monoclonal antibody of which a detection signal was positively correlated with the toxigenicity of the Aspergillus flavus strain, i.e., an antibody of indicator molecule for toxigenicity of Aspergillus flavus strain, was found out, and a protein corresponding to the indicator molecule for toxigenicity of Aspergillus flavus strain was the discovered indicator molecule for toxigenicity of Aspergillus flavus strain.

In the above solution, the *Aspergillus flavus* strain with high toxigenicity in the step (1) was isolated and identified from the nature by a conventional method, or obtained by artificial engineering, and its toxigenicity was identified by a standard method of NY/T 2311-2013 to be not less than 10 µg/kg.

There were at least three Aspergillus flavus strains with different toxigenicity in the step (3), and the toxigenicity thereof were identified by the standard method of NY/T 2311-2013, showing at least three levels: high, medium, and low.

A medium used in the culture of the *Aspergillus flavus* strain with high toxigenicity was a Czapek medium or other nutrients for normal growth of Aspergillus flavus, and culture was performed for a period of not less than 12 hours at an ambient temperature of 15-35°C.

The cells of the strain culture were disrupted by methods such as conventional liquid nitrogen grinding or a cell disrupter.

The carbodiimide was used in an amount of 0.005-0.1 g per 1.0 mL of the mixture of the extracellular secreted protein mixture and the intracellular protein mixture.

The coupling reaction refers to a reaction for 2-6 h at 15-37°C and a reaction overnight at 4-10°C.

The immunization is a conventional immunization manner, with vaccination with the Aspergillus flavus antigen. The test animal is a mouse or an alpaca or other test animals having a similar effect.

According to the above solution, the antibody preparation process refers to a conventional nanobody preparation technique process or a conventional cell fusion-based hybridoma monoclonal antibody preparation technique process.

According to the above solution, the detection of the proteins of the Aspergillus flavus strains having different toxigenicity refers to detection using the conventional Western Blot technical process, i.e., transferring the proteins of the Aspergillus flavus strains having different toxigenicity onto a nitrocellulose membrane, and then using a direct or indirect method by using antibodies from the antibody library, or using other technical processes with similar efficacy.

According to the above solution, the direct method means that the antibodies from the antibody library are coupled to a signal material by conventional methods, and the obtained conjugate immunobinds to the corresponding proteins transferred onto the nitrocellulose membrane.

According to the above solution, the indirect method means that the antibodies from the antibody library immunobind to the corresponding proteins transferred onto the nitrocellulose membrane, and then a conjugate of a second antibody and a signal material immunobinds to the antibodies bound to the nitrocellulose membrane.

In the above detection, the signal material is horseradish peroxidase or colloidal gold or a fluorescent material or other materials with similar efficacy. The detection signal is a chromogenic reaction signal or a spot signal or a fluorescent signal.

### Example 2 Preparation of nanobody against the molecule AFT-YJFZP008 indicating the toxigenicity of the aflatoxin-producing fungi

The nanobodies can be developed by immunizing alpacas or Balb/c mice in a conventional manner by using AFT-YJFZ01 as an immunizing antigen, and then using a known conventional nanobody or murine monoclonal antibody preparation technical solution.

The prepared AFT-YJFZ01 was dissolved in a conventional PBS buffer or physiological saline to a concentration of not less than 0.1 mg/mL, the obtained solution was mixed with a Freund's complete adjuvant in an equal volume to be emulsified, and the alpacas were immunized by multipoint injection subcutaneously or intradermally in the backs, followed by booster immunization once every other 2-4 weeks, wherein the Freund's complete adjuvant was replaced with a Freund's incomplete adjuvant during the booster immunization. Immune effects were monitored by using a conventional ELISA process until a serum titer of the alpacas no longer rose, and subsequently, operations such as collection of venous blood of the immunized alpacas, total RNA extraction, cDNA synthesis, VHH gene amplification, VHH gene fragment recovery, ligation of a VHH gene to a pCANTAB 5 E (his) vector treated by double enzyme digestion, electro-transformation of a ligation product, construction of a nanobody gene bank and rescue of the nanobody gene bank were completed with reference to the methods in a patent document CN103866401A, so that the rescued nanobody gene bank was finally obtained.

The prepared AFT-YJFZ01 was immobilized on a solid support such as a 96-well ELISA plate at a gradient of 8 µg/well, 2 µg/well, 0.5 µg/well, and 0.1 µg/well, the above rescued nanobody gene bank was subjected to panning for 2-4 times with reference to the method in the patent document CN103866401A, antibodies produced by each phage clone were then identified with AFT-YJFZ01 and indirect non-competitive ELISA, a phage corresponding to a positive result was a phage positive clone, and the positive clone was used to prepare a nanobody, i.e. the nanobody of AFT-YJFZ01, in a conventional manner of nanobody preparation for further research work, preferably a nanobody with high specificity and high affinity, characterized by ELISA.

### Example 3 Preparation of monoclonal antibodies against the molecule AFT-YJFZP008 indicating the toxigenicity of the aflatoxin-producing fungi

The monoclonal antibodies can be developed by immunizing alpacas or Balb/c mice in a conventional manner by using AFT-YJFZ01 as an immunizing antigen, and then using a known conventional nanobody or murine monoclonal antibody preparation technical solution.

The prepared AFT-YJFZ01 was dissolved in a conventional PBS buffer or physiological saline to a concentration of not less than 0.1 mg/mL, the obtained solution was mixed with a Freund's complete adjuvant in an equal volume to be emulsified, and the Balb/c mice were immunized by multipoint injection subcutaneously or intradermally in the backs, followed by booster immunization once every other 2-4 weeks, wherein the Freund's complete adjuvant was replaced with a Freund's incomplete adjuvant during the booster immunization. Immune effects were monitored by using a conventional ELISA process until a serum titer of the Balb/c mice no longer rose, subsequently, operations of isolation of splenocytes of the immunized mice, fusion of the splenocytes with murine myeloma cells SP2/0, and selective culture of hybridoma cells with a semi-solid medium were completed by referring to the methods in a patent document CN103849604A, and after acicular white spots grew on the semi-solid medium, the white spots were individually picked into a 96-well culture plate in which a conventional medium for hybridomas was built in, thereby obtaining a monoclonal hybridoma resource library.

A culture supernatant of the above monoclonal hybridoma, that is, the monoclonal antibody, was obtained by referring to the method in the patent document CN103849604A, the prepared AFT-YJFZ01 was immobilized on a solid support such as a 96-well ELISA plate at a gradient of 8 µg/well, 2 µg/well, 0.5 µg/well, and 0.1 µg/well, each monoclonal antibody was then identified by an indirect non-competitive ELISA procedure, and positive clones were picked to obtain the monoclonal antibody of AFT-YJFZ01 for further research work, preferably the monoclonal antibody of AFT-YJFZ01 with high specificity and high affinity after detection.

### Example 4 Preparation of rabbit-derived polyclonal antibodies against the molecule AFT-YJFZP008 indicating the toxigenicity of the aflatoxin-producing fungi

The rabbit-derived polyclonal antibodies can be developed by immunizing test rabbits such as New Zealand white rabbits in a conventional manner by using AFT-YJFZ01 as an immunizing antigen, and then using a known conventional rabbit polyclonal antibody preparation technical solution.

The AFT-YJFZP008 prepared above was directly used as an antigen, a solution of AFT-YJFZ01 with a concentration of not less than 0.1 mg/mL was mixed with a Freund's complete adjuvant in an equal volume to be emulsified, and the New Zealand white rabbits were immunized by multipoint injection subcutaneously or intradermally in the backs, followed by booster immunization once every other 2-4 weeks, wherein the Freund's complete adjuvant was replaced with a Freund's incomplete adjuvant during the booster immunization. Immune effects were monitored by using a conventional ELISA process until a serum titer of the immunized animals no longer rose, and the serum of the immunized animals, that is, the rabbit-derived polyclonal antibodies against the molecule AFT-YJFZP008 indicating the toxigenicity of the aflatoxin-producing fungi, was prepared by conventional methods.

### Example 5 Identification of toxigenicity of aflatoxin-producing strain by using the molecule AFT-YJFZP008 indicating the toxigenicity of the aflatoxin-producing fungi

Step 1, Preparation of to-be-tested solutions of strains to be identified: ten published toxigenic strains, including HBZHX-21, HBXY-36, HBHA-1-4, GDZJ-6, HeNZY-2, HuBha-24, JSnt-1, HuNdx-7, GDZJ-108-19, and HBHA-8-17 were selected from Page 33 of the published literature "Study on Distribution, Toxigenicity and Infection of Aspergillus flavus in Typical Peanut Producing Areas of China"--Master's Thesis of Chinese Academy of Agricultural Sciences, of which the author is Zhang Xing according to the toxigenicity of the strains, the strains to be identified were cultured in a conventional Czapek medium or other media suitable for the growth of Aspergillus flavus producing toxins at an ambient temperature of 15-35°C for a period of not less than 12 hours, a mixture of the medium and a culture was well homogenized, and diluted 5-fold with sterile water to obtain the to-be-tested solutions of the strains to be identified.

Step 2, Determination of the to-be-tested solutions of the strains to be identified: the nanobody or monoclonal antibody of AFT-YJFZ01 was dissolved in a conventional ELISA coating buffer to form a coating solution of 2 µg/mL, and the coating solution was added into a 96-well ELISA plate at 200 µL/well; after standing at 4°C overnight or at 37°C for not less than 2h, the coating solution was removed from the ELISA plate, and the ELISA plate was washed with a conventional washing solution for ELISA; then, skimmed milk powder having a concentration of not less than 1% was used as a blocking solution, 300 µL of the blocking solution was added into each well, and after blocking for not less than 1h at room temperature or 37°C, the blocking solution was discarded, and the ELISA plate was washed with the conventional washing solution for ELISA; the above to-be-tested solutions were then appropriately diluted with a conventional phosphate buffer with a pH close to be neutral, and 200 µL of the diluted to-be-tested solutions were added into each well or 200 µL of solutions of the toxigenicity indicator molecule AFT-YJFZ01 of the present disclosure with serial concentrations were added, and after blocking at room temperature or 37°C for not less than 1h, a liquid was discarded, and the ELISA plate was washed with the conventional washing solution for ELISA; then, the rabbit-derived polyclonal antibody of AFT-YJFZ01 was appropriately diluted with a conventional phosphate buffer with a pH close to be neutral, and 200 µL of the diluted rabbit-derived polyclonal antibody was added into each well, and after blocking at room temperature or 37°C for not less than 1h, a liquid was discarded, and the ELISA plate was washed with the conventional washing solution for ELISA; then, a commercial horseradish peroxidase labeled goat anti-rabbit antibody was diluted with a conventional phosphate buffer with a pH close to be neutral according to the instructions, 200 µL of the diluted commercial horseradish peroxidase labeled goat anti-rabbit antibody was added, and after blocking at room temperature or 37°C for not less than 1h, a liquid was discarded, and the ELISA plate was washed with the conventional washing solution for ELISA; and then, a conventional chromogen solution for ELISA and a stop solution were sequentially added, and finally reading was performed by a microplate reader, and the content of AFT-YJFZ01 was calculated.

According to the results of the above-mentioned literature "Study on Distribution, Toxigenicity and Infection of Aspergillus flavus in Typical Peanut Producing Areas of China", the toxigenicity of the ten strains HBZHX-21, HBXY-36, HBHA-1-4, GDZJ-6, HeNZY-2, HuBha-24, JSnt-1, HuNdx-7, GDZJ-108-19, and HBHA-8-17 was 0 µg/L, 0 µg/L, 3.8 µg/L, 4.9 µg/L, 67.2 µg/L, 81.7 µg/L, 192.0 µg/L, 204.4 µg/L, 297.4 µg/L, and 1027.5 µg/L in sequence, indicating that HBZHX-21 and HBXY-36 are aflatoxin-non-producing strains, HBHA-1-4 and GDZJ-6, etc. are aflatoxin- producing strains with low toxigenicity, and HBHA-8-17 and GDZJ-108-19, etc. are aflatoxin- producing strains with high toxigenicity.

Step 3, Evaluation of identification results: the higher the content of the toxigenicity indicator molecule AFT-YJFZ01 in the strains to be identified, the stronger the aflatoxin-producing ability, that is, the toxigenicity, of the strains to be identified. The results of the content of AFT-YJFZ01 in the ten strains HBZHX-21, HBXY-36, HBHA-1-4, GDZJ-6, HeNZY-2, HuBha-24, JSnt-1, HuNdx-7, GDZJ-108-19, and HBHA-8-17 determined according to the above technical solution: the content of AFT-YJFZ01 in each milliliter of the medium was 0 ng, 0 ng, 8 ng, 12 ng, 49 ng, 51 ng, 104 ng, 115 ng, 175 ng, and 536 ng in sequence also showed that HBZHX-21 and HBXY-36 are aflatoxin-non-producing strains, HBHA-1-4 and GDZJ-6, etc. are aflatoxin-producing strains with low toxigenicity, and HBHA-8-17 and GDZJ-108-19, etc. are aflatoxin-producing strains with high toxigenicity. The determination results are consistent with the order of the toxigenicity of the strains published in the above-mentioned published literature "Study on Distribution, Toxigenicity and Infection of Aspergillus flavus in Typical Peanut Producing Areas of China", and the results of identification of aflatoxin- producing strains with high toxigenicity and aflatoxin- producing strains with low toxigenicity are consistent with those in the literature, demonstrating that the technical solution provided by the present disclosure can be used to identify the toxigenicity of aflatoxin-producing strains, and the method is simple, easy to operate, and high in practicality.

### Example 6 Identifying whether there is an aflatoxin- producing strain with high toxigenicity in agricultural fields by using indicator molecule AFT-YJFZ01

Step 1, Preparation of to-be-tested solutions of samples to be identified: 4 rhizosphere soil samples of peanuts in a flowering stage in Jilin, Liaoning, Jiangxi, Fujian, etc. were selected, and named as a soil sample 1, a soil sample 2, a soil sample 3, and a soil sample 4 in sequence. Agricultural field soil samples to be tested were weighed in sequence, crushed and transferred to sterile water to be a concentration of 0.5 g/mL, and the obtained solution was shaken at room temperature until the solution was uniform to prepare uniform dispersions of the samples to be tested. 50 µL of the uniform dispersion of the aboved soil was added to 30 mL of a conventional liquid Sabouraud medium to be incubated at 28°C with shaking at 200 rpm for 24 h, and samples were taken to form the to-be-tested solutions of the samples to be identified.

Step 2, Determination of the to-be-tested solutions of the samples to be identified: the nanobody or monoclonal antibody of AFT-YJFZ01 was dissolved in a conventional ELISA coating buffer to form a coating solution of 0.2-8.0 µg/mL, and the coating solution was added into a 96-well ELISA plate at 200 µL/well; after standing at 4°C overnight or at 37°C for not less than 2h, the coating solution was removed from the ELISA plate, and the ELISA plate was washed with a conventional washing solution for ELISA; then, skimmed milk powder having a concentration of not less than 1% was used as a blocking solution, 300 µL of the blocking solution was added into each well, and after blocking for not less than 1h at room temperature or 37°C, the blocking solution was discarded, and the ELISA plate was washed with the conventional washing solution for ELISA; the above to-be-tested solutions were then appropriately diluted with a conventional phosphate buffer with a pH close to be neutral, and 200 µL of the diluted to-be-tested solutions were added into each well or 200 µL of solutions of the indicator molecule AFT-YJFZ01 of the present disclosure with serial concentrations (0.00003, 0.0003, 0.003, 0.03, 0.3, 3, 30, and 300 ng/mL, for obtaining a standard curve) were added, and after blocking at room temperature or 37°C for not less than 1h, a liquid was discarded, and the ELISA plate was washed with the conventional washing solution for ELISA; then, the rabbit-derived polyclonal antibody of AFT-YJFZ01 was appropriately diluted with a conventional phosphate buffer with a pH close to be neutral, and 200 µL of the diluted rabbit-derived polyclonal antibody was added into each well, and after blocking at room temperature or 37°C for not less than 1h, a liquid was discarded, and the ELISA plate was washed with the conventional washing solution for ELISA; then, a commercial horseradish peroxidase labeled goat anti-rabbit antibody was diluted with a conventional phosphate buffer with a pH close to be neutral according to the instructions, 200 µL of the diluted commercial horseradish peroxidase labeled goat anti-rabbit antibody was added, and after blocking at room temperature or 37°C for not less than 1h, a liquid was discarded, and the ELISA plate was washed with the conventional washing solution for ELISA; and then, a conventional chromogen solution for ELISA and a stop solution were sequentially added, and finally reading was performed by a microplate reader, and the content result of AFT-YJFZ01 was calculated based on the standard curve.

Step 3, Evaluation of identification results: if the content of the indicator molecule AFT-YJFZ01 in the samples to be identified was high, it indicated that the samples to be identified contained a highly virulent aflatoxin-producing strain. The results of the content of AFT-YJFZ01 in the soil sample 1, the soil sample 2, the soil sample 3, and the soil sample 4 determined according to the above technical solution were as follows: the content of AFT-YJFZ01 in each milliliter of culture solutions was 0.3 ng, 0.2 ng, 16 ng, and 19 ng in sequence. The results show that the content of AFT-YJFZ01 in each milliliter of culture solutions of the soil sample 1 and the soil sample 2 was 1.0 ng or less, the soil sample 1 and the soil sample 2 did not contain a highly virulent aflatoxin-producing strain, and peanuts in their corresponding agricultural fields have a low risk of pollution after production; and the content of AFT-YJFZ01 in each milliliter of culture solutions of the soil sample 3 and the soil sample 4 was 10 ng or more, the soil sample 3 and the soil sample 4 contained **an** aflatoxin- producing strain with high toxigenicity, and peanuts in their corresponding agricultural fields have a higher risk of pollution after production.

### Example 7 Identifying whether there is an aflatoxin- producing strain with high toxigenicity in agricultural products by using indicator molecule AFT-YJFZ01

Step 1, Preparation of to-be-tested solutions of samples to be identified, 4 samples of agricultural products such as peanut, corn, rice, wheat and the like were selected, and the agricultural product samples to be tested were weighed in sequence, crushed and transferred to sterile water to be a concentration of 0.5 g/mL, and the obtained solution was shaken at room temperature until the solution was uniform to prepare uniform dispersions of the samples to be tested. 100 µL of the uniform dispersion of each sample was added to 50 mL of a conventional liquid Sabouraud medium to be incubated at 28°C with shaking at 200 rpm for 6h, and samples were taken to form the to-be-tested solutions of the samples to be identified.

Step 2, Determination of the to-be-tested solutions of the samples to be identified: the nanobody or monoclonal antibody of AFT-YJFZ01 was dissolved in a conventional ELISA coating buffer to form a coating solution of 0.2-8.0 µg/mL, and the coating solution was added into a 96-well ELISA plate at 200 µL/well; after standing at 4°C overnight or at 37°C for not less than 2h, the coating solution was removed from the ELISA plate, and the ELISA plate was washed with a conventional washing solution for ELISA; then, skimmed milk powder having a concentration of not less than 1% was used as a blocking solution, 300 µL of the blocking solution was added into each well, and after blocking for not less than 1h at room temperature or 37°C, the blocking solution was discarded, and the ELISA plate was washed with the conventional washing solution for ELISA; the above to-be-tested solutions were then appropriately diluted with a conventional phosphate buffer with a pH close to be neutral, and 200 µL of the diluted to-be-tested solutions were added into each well or 200 µL of solutions of the indicator molecule AFT-YJFZ01 of the present disclosure with serial concentrations (0.00003, 0.0003, 0.003, 0.03, 0.3, 3, 30, and 300 ng/mL, for obtaining a standard curve) were added, and after blocking at room temperature or 37°C for not less than 1h, a liquid was discarded, and the ELISA plate was washed with the conventional washing solution for ELISA; then, the rabbit-derived polyclonal antibody of AFT-YJFZ01 was appropriately diluted with a conventional phosphate buffer with a pH close to be neutral, and 200 µL of the diluted rabbit-derived polyclonal antibody was added into each well, and after blocking at room temperature or 37°C for not less than 1h, a liquid was discarded, and the ELISA plate was washed with the conventional washing solution for ELISA; then, a commercial horseradish peroxidase labeled goat anti-rabbit antibody was diluted with a conventional phosphate buffer with a pH close to be neutral according to the instructions, 200 µL of the diluted commercial horseradish peroxidase labeled goat anti-rabbit antibody was added, and after blocking at room temperature or 37°C for not less than 1h, a liquid was discarded, and the ELISA plate was washed with the conventional washing solution for ELISA; and then, a conventional chromogen solution for ELISA and a stop solution were sequentially added, and finally reading was performed by a microplate reader, and the content result of AFT-YJFZ01 was calculated based on the standard curve.

Step 3, Evaluation of identification results: if the content of the indicator molecule AFT-YJFZ01 in the samples to be identified was high, it indicated that the samples to be identified contained an aflatoxin- producing strain with high toxigenicity. The results of the content of AFT-YJFZ01 in peanut, corn, rice and wheat samples determined according to the above technical solution were as follows: the content of AFT-YJFZ01 in each milliliter of culture solutions was 12 ng, 13 ng, 0.7 ng, and 0 ng in sequence. The results show that the content of AFT-YJFZ01 in each milliliter of the culture solutions of the peanut and corn samples was 10 ng or more, and the peanut and corn samples contained an aflatoxin- producing strain with high toxigenicity, with a higher risk of pollution; and the content of AFT-YJFZ01 in each milliliter of the culture solution of the rice sample was 1.0 or less, and the rice sample did not contain an aflatoxin- producing strain with high toxigenicity, with a low risk of pollution; and the content of AFT-YJFZ01 in each milliliter of the culture solution of the wheat sample determined was 0, and the wheat sample did not contain aflatoxin-producing strains, essentially without a risk of aflatoxin pollution.

### Example 8 Identifying whether there is an aflatoxin- producing strain with high toxigenicity in feed by using indicator molecule AFT-YJFZ01

Step 1, Preparation of to-be-tested solutions of samples to be identified: a total of 4 feed samples to be tested were selected from the market, and named as a feed 1, a feed 2, a feed 3, and a feed 4 in sequence. The feed samples to be tested were weighed in sequence, crushed and transferred to sterile water to be a concentration of 0.5 g/mL, and the obtained solution was shaken at room temperature until the solution was uniform to prepare uniform dispersions of the samples to be tested. 50 µL of the uniform dispersion of each sample was added to 30 mL of a conventional liquid Sabouraud medium to be incubated at 28°C with shaking at 200 rpm for 24h, and samples were taken to form the to-be-tested solutions of the samples to be identified.

Step 2, Determination of the to-be-tested solutions of the samples to be identified: the nanobody or monoclonal antibody of AFT-YJFZ01 was dissolved in a conventional ELISA coating buffer to form a coating solution of 0.2-8.0 µg/mL, and the coating solution was added into a 96-well ELISA plate at 200 µL/well; after standing at 4°C overnight or at 37°C for not less than 2h, the coating solution was removed from the ELISA plate, and the ELISA plate was washed with a conventional washing solution for ELISA; then, skimmed milk powder having a concentration of not less than 1% was used as a blocking solution, 300 µL of the blocking solution was added into each well, and after blocking for not less than 1h at room temperature or 37°C, the blocking solution was discarded, and the ELISA plate was washed with the conventional washing solution for ELISA; the above to-be-tested solutions were then appropriately diluted with a conventional phosphate buffer with a pH close to be neutral, and 200 µL of the diluted to-be-tested solutions were added into each well or 200 µL of solutions of the indicator molecule AFT-YJFZ01 of the present disclosure with serial concentrations (0.00003, 0.0003, 0.003, 0.03, 0.3, 3, 30, and 300 ng/mL, for obtaining a standard curve) were added, and after blocking at room temperature or 37°C for not less than 1h, a liquid was discarded, and the ELISA plate was washed with the conventional washing solution for ELISA; then, the rabbit-derived polyclonal antibody of AFT-YJFZ01 was appropriately diluted with a conventional phosphate buffer with a pH close to be neutral, and 200 µL of the diluted rabbit-derived polyclonal antibody was added into each well, and after blocking at room temperature or 37 C for not less than 1h, a liquid was discarded, and the ELISA plate was washed with the conventional washing solution for ELISA; then, a commercial horseradish peroxidase labeled goat anti-rabbit antibody was diluted with a conventional phosphate buffer with a pH close to be neutral according to the instructions, 200 µL of the diluted commercial horseradish peroxidase labeled goat anti-rabbit antibody was added, and after blocking at room temperature or 37°C for not less than 1h, a liquid was discarded, and the ELISA plate was washed with the conventional washing solution for ELISA; and then, a conventional chromogen solution for ELISA and a stop solution were sequentially added, and finally reading was performed by a microplate reader, and the content result of AFT-YJFZ01 was calculated.

Step 3, Evaluation of identification results: if the content of the indicator molecule AFT-YJFZ01 in the samples to be identified was high, it indicated that the samples to be identified contained a highly virulent aflatoxin-producing strain. The results of the content of AFT-YJFZ01 in the feed 1, the feed 2, the feed 3 and the feed 4 determined according to the above technical solution were as follows: the content of AFT-YJFZ01 in each milliliter of culture solutions was 0.6 ng, 22 ng, 13 ng, and 69 ng in sequence. The results show that the content of AFT-YJFZ01 in each milliliter of the culture solution of the feed 1 was 1.0 ng or less, and the feed 1 did not contain an aflatoxin- producing strain with high toxigenicity, with a low risk of pollution; and taking the condition that 50 µL of the uniform dispersion of each sample was added into 30 mL of the medium as an example, the content of AFT-YJFZ01 in each milliliter of the culture solutions of the feed 2, the feed 3, and the feed 4 was 10 ng or more, and thus, the feed 2, the feed 3, and the feed 4 contained an aflatoxin- producing strain with high toxigenicity.

## Claims

1. An indicator molecule AFT-YJFZ01 for toxigenicity of aflatoxigenic fungi, having the amino acid sequence shown in SEQ ID NO. 1.

2. A method for identifying toxigenicity of aflatoxigenic fungi in a system, comprising detecting a content of an indicator molecule AFT-YJFZ01 for toxigenicity of aflatoxigenic fungi, and identifying the toxigenicity of aflatoxigenic fungi in a system on the basis of the content of the indicator molecule AFT-YJFZ01 for toxigenicity of aflatoxigenic fungi, the indicator molecule AFT-YJFZ01 for toxigenicity of aflatoxigenic fungi having the amino acid sequence shown in SEQ ID NO. 1.

3. A method for identifying an aflatoxin-producing ability of aflatoxin-producing strains of genus *Aspergillus* using the method according to claim 2, comprising:
(1) providing a nanobody or monoclonal antibody of the indicator molecule AFT-YJFZ01 for toxigenicity of aflatoxigenic fungi;
(2) providing a polyclonal antibody of the indicator molecule AFT-YJFZ01 for toxigenicity of aflatoxigenic fungi;
(3) preparation of a to-be-tested solution of a strain to be identified: culturing the strain to be identified, and diluting to obtain the to-be-tested solution of the strain to be identified; and
(4) determination of the aflatoxin-producing ability of the strain to be identified:
identifying the aflatoxin-producing ability of the aflatoxin-producing strains of genus *Aspergillus* by using an indirect non-competitive double antibody sandwich method, comprising the steps of: coating an ELISA plate with the nanobody or monoclonal antibody of AFT-YJFZ01, and washing the plate;
performing blocking by adding a blocking solution, and washing the plate;
adding the to-be-tested solution, carrying out a reaction, and washing the plate;
adding the polyclonal antibody of AFT-YJFZ01, carrying out a reaction, and washing the plate;
adding a horseradish peroxidase labeled antibody that binds to the polyclonal antibody of the indicator molecule AFT-YJFZ01 for toxigenicity of aflatoxigenic fungi, carrying out a reaction, and washing the plate;
adding a chromogen solution, and carrying out a reaction; and
adding a stop solution, performing reading by using a microplate reader, and performing calculation to obtain a content of the indicator molecule AFT-YJFZ01 for toxigenicity of aflatoxigenic fungi, wherein the higher the content of the indicator molecule AFT-YJFZ01 for toxigenicity of aflatoxigenic fungi in the to-be-tested solution of the strain to be identified, the stronger the aflatoxin-producing ability of the aflatoxin-producing strain; and
the indicator molecule AFT-YJFZ01 for toxigenicity of aflatoxigenic fungi has the amino acid sequence shown in SEQ ID NO 1.

4. The method according to claim 3, wherein the step (3) comprises culturing the strain to be identified in a conventional Czapek medium or other media suitable for growth of strains at an ambient temperature of 15-35°C for not less than 12 hours, taking a mixture of the medium and a culture to be well homogenized, and performing diluting by 1-10 times with sterile water to obtain the to-be-tested solution of the strain to be identified.

5. The method according to claim 3, wherein the step (4) comprises:
Step a: preparing a coating solution of 0.2-8.0 µg/mL from the nanobody or monoclonal antibody of AFT-YJFZ01 by using an ELISA coating buffer, adding the coating solution into the ELISA plate, removing the coating solution from the ELISA plate after standing overnight at 4°C or at 37°C for not less than 2h, and washing the ELISA plate with a conventional washing solution for ELISA; and then performing blocking at room temperature or 37°C for not less than 1h by using skimmed milk powder having a concentration of not less than 1% as the blocking solution, discarding the blocking solution, and washing the ELISA plate with the conventional washing solution for ELISA;
Step b: then appropriately diluting the to-be-tested solution of the strain to be identified with a conventional phosphate buffer with a pH close to be neutral, adding the diluted to-be-tested solution to wells of the ELISA plate, performing blocking at room temperature or 37°C for not less than 1h, discarding a liquid, and washing the ELISA plate with the conventional washing solution for ELISA;
Step c: then appropriately diluting the polyclonal antibody of AFT-YJFZ01 with a conventional phosphate buffer with a pH close to be neutral, adding the diluted polyclonal antibody into wells of the ELISA plate, performing blocking at room temperature or 37°C for not less than 1h, discarding a liquid, and washing the ELISA plate with the conventional washing solution for ELISA;
Step d: then diluting a commercial horseradish peroxidase labeled antibody with a conventional phosphate buffer with a pH close to be neutral as needed, adding the diluted commercial horseradish peroxidase labeled antibody into wells of the ELISA plate, performing blocking at room temperature or 37°C for not less than 1h, discarding a liquid, and washing the ELISA plate with the conventional washing solution for ELISA; and
Step e: then sequentially adding a conventional chromogen solution for ELISA and the stop solution, and finally reading by using the microplate reader, and calculating a content result of AFT-YJFZ01.

6. A method for identifying whether there is an aflatoxin-producing strain with high toxigenicity in a sample using the method according to claim 2, specifically comprising the following steps:
(1) providing a nanobody or monoclonal antibody of the indicator molecule AFT-YJFZ01 for toxigenicity of aflatoxigenic fungi
(2) providing a polyclonal antibody of the indicator molecule AFT-YJFZ01 for toxigenicity of aflatoxigenic fungi;
(3) preparation of a to-be-tested solution of a sample to be identified: culturing the sample to be identified, and diluting to obtain the to-be-tested solution of the sample to be identified;
(4) identifying whether there is an aflatoxin-producing strain with high toxigenicity in a sample: identifying the sample by using an indirect non-competitive double antibody sandwich method, comprising the steps of:
coating an ELISA plate with the nanobody or monoclonal antibody of AFT-YJFZ01, and washing the plate;
performing blocking by adding a blocking solution, and washing the plate;
adding the to-be-tested solution of the sample to be identified, carrying out a reaction, and washing the plate;
adding the polyclonal antibody of AFT-YJFZ01, carrying out a reaction, and washing the plate;
adding a horseradish peroxidase labeled antibody that binds to the polyclonal antibody of the indicator molecule AFT-YJFZ01, carrying out a reaction, and washing the plate;
adding a chromogen solution, and carrying out a reaction; and
adding a stop solution, performing reading by using a microplate reader, and performing calculation to obtain a content of the indicator molecule AFT-YJFZ01 for toxigenicity of aflatoxigenic fungi; and
(5) evaluation of identification results:
the higher the content of the indicator molecule AFT-YJFZ01 for toxigenicity of aflatoxigenic fungi in the sample to be identified, the stronger the aflatoxin-producing ability, that is, the toxigenicity, of the sample to be identified, obtaining the content of the indicator molecule AFT-YJFZ01 for toxigenicity of aflatoxigenic fungi in the to-be-tested solution of the sample per unit volume based on a result of calculation by the microplate reader, and determining whether the aflatoxin-producing strain with high toxigenicity is contained in the sample to be identified; wherein
the indicator molecule AFT-YJFZ01 for toxigenicity of aflatoxigenic fungi has the amino acid sequence shown in SEQ ID NO 1.

7. The method according to claim 6, wherein the step (3) comprises: weighing the sample to be identified, transferring the weighed sample to be identified to sterile water, performing shaking at room temperature until the obtained solution is uniform to prepare a uniform dispersion of the sample to be tested, taking 10-1000 µL of the uniform dispersion of the sample to be added into 6-600 mL of a conventional Czapek medium or other media suitable for growth of Aspergillus flavus producing toxins, performing shaking culture at 15-35°C at 200±50 rpm after 6-24 h, and taking a sample to form the to-be-tested solution of the sample to be identified; wherein the sample to be identified is soil, a traditional Chinese medicine, an agricultural product or a feed.

8. The method according to claim 6, wherein the step (4) specifically comprises:
Step a: preparing a coating solution of 0.2-8.0 µg/mL from the nanobody or monoclonal antibody of AFT-YJFZ01 by using an ELISA coating buffer, adding the coating solution into the ELISA plate, removing the coating solution from the ELISA plate after standing overnight at 4°C or at 37°C for not less than 2h, and washing the ELISA plate with a conventional washing solution for ELISA; and then performing blocking at room temperature or 37°C for not less than 1h by using skimmed milk powder having a concentration of not less than 1% as the blocking solution, discarding the blocking solution, and washing the ELISA plate with the conventional washing solution for ELISA;
Step b: then appropriately diluting the to-be-tested solution of the sample to be identified with a conventional phosphate buffer with a pH close to be neutral, adding the diluted to-be-tested solution to wells of the ELISA plate, performing blocking at room temperature or 37°C for not less than 1h, discarding a liquid, and washing the ELISA plate with the conventional washing solution for ELISA;
Step c: then appropriately diluting the polyclonal antibody of AFT-YJFZ01 with a conventional phosphate buffer with a pH close to be neutral, adding the diluted polyclonal antibody into wells of the ELISA plate, performing blocking at room temperature or 37°C for not less than 1h, discarding a liquid, and washing the ELISA plate with the conventional washing solution for ELISA;
Step d: then diluting a commercial horseradish peroxidase labeled antibody with a conventional phosphate buffer with a pH close to be neutral as needed, adding the diluted commercial horseradish peroxidase labeled antibody into wells of the ELISA plate, performing blocking at room temperature or 37°C for not less than 1h, discarding a liquid, and washing the ELISA plate with the conventional washing solution for ELISA; and
Step e: then sequentially adding a conventional chromogen solution for ELISA and the stop solution, and finally reading by using the microplate reader, and calculating a content result of AFT-YJFZ01.

9. The method according to claim 3 or 6, wherein an animal source of the polyclonal antibody of the indicator molecule AFT-YJFZ01 for toxigenicity of aflatoxigenic fungi is different from an animal source of the nanobody or monoclonal antibody of the indicator molecule AFT-YJFZ01 for toxigenicity of aflatoxigenic fungi.

10. The method according to claim 3 or 6, wherein solutions of the indicator molecule AFT-YJFZ01 for toxigenicity of aflatoxigenic fungi with a series of concentration gradients are used to make a standard curve, and the content of AFT-YJFZ01 is obtained by using the standard curve based on the results of the microplate reader.

## Patentansprüche

1. Indikatormolekül AFT-YJFZ01 für die Toxigenität aflatoxigener Pilze mit der in SEQ ID NO. 1 gezeigten Aminosäuresequenz.

2. Verfahren zur Identifizierung der Toxigenität aflatoxigener Pilze in einem System, umfassend das Nachweisen eines Gehalts eines Indikatormoleküls AFT-YJFZ01 für die Toxigenität aflatoxigener Pilze und das Identifizieren der Toxigenität aflatoxigener Pilze in einem System auf der Grundlage des Gehalts des Indikatormoleküls AFT-YJFZ01 für die Toxigenität aflatoxigener Pilze, wobei das Indikatormolekül AFT-YJFZ01 für die Toxigenität aflatoxigener Pilze die in SEQ ID NO. 1 gezeigte Aminosäuresequenz aufweist.

3. Verfahren zur Identifizierung einer Aflatoxin-Produktionsfähigkeit von Aflatoxin produzierenden Stämmen der Gattung *Aspergillus* unter Verwendung des Verfahrens nach Anspruch 2, umfassend:
(1) Bereitstellen eines Nanobodys oder monoklonalen Antikörpers des Indikatormoleküls AFT-YJFZ01 für die Toxigenität aflatoxigener Pilze;
(2) Bereitstellen eines polyklonalen Antikörpers des Indikatormoleküls AFT-YJFZ01 für die Toxigenität aflatoxigener Pilze;
(3) Herstellen einer zu testenden Lösung eines zu identifizierenden Stamms: Kultivieren des zu identifizierenden Stamms und Verdünnen, um die zu testende Lösung des zu identifizierenden Stamms zu erhalten, und
(4) Bestimmen der Aflatoxin-Produktionsfähigkeit des zu identifizierenden Stamms:
Identifizieren der Aflatoxin-Produktionsfähigkeit der Aflatoxin produzierenden Stämme der Gattung *Aspergillus* unter Verwendung eines indirekten nicht-kompetitiven Doppelantikörper-Sandwich-Verfahrens, das die folgenden Schritte umfasst: Beschichten einer ELISA-Platte mit dem Nanobody oder monoklonalen Antikörper von AFT-YJFZ01 und Waschen der Platte;
Durchführen einer Blockierung durch Zugeben einer Blockierungslösung und Waschen der Platte;
Zugeben der zu testenden Lösung, Ausführen einer Reaktion und Waschen der Platte;
Zugeben des polyklonalen Antikörpers von AFT-YJFZ01, Ausführen einer Reaktion und Waschen der Platte;
Zugeben eines mit Meerrettichperoxidase markierten Antikörpers, der an den polyklonalen Antikörper des Indikatormoleküls AFT-YJFZ01 für die Toxigenität aflatoxigener Pilze bindet, Ausführen einer Reaktion und Waschen der Platte;
Zugeben einer Chromogenlösung und Ausführen einer Reaktion und
Zugeben einer Stopplösung, Durchführen einer Ablesung unter Verwendung eines Mikroplatten-Lesegeräts und Durchführen einer Berechnung, um einen Gehalt des Indikatormoleküls AFT-YJFZ01 für die Toxigenität aflatoxigener Pilze zu erhalten, wobei, je höher der Gehalt des Indikatormoleküls AFT-YJFZ01 für die Toxigenität aflatoxigener Pilze in der zu testenden Lösung des zu identifizierenden Stamms ist, desto stärker die Aflatoxin-Produktionsfähigkeit des Aflatoxin produzierenden Stamms ist, und
das Indikatormolekül AFT-YJFZ01 für die Toxigenität aflatoxigener Pilze die in SEQ ID NO. 1 gezeigte Aminosäuresequenz aufweist.

4. Verfahren nach Anspruch 3, wobei Schritt (3) das Kultivieren des zu identifizierenden Stamms in einem konventionellen Czapek-Medium oder anderen Medien, die für das Wachstum von Stämmen geeignet sind, bei einer Umgebungstemperatur von 15-35 °C für nicht weniger als 12 Stunden, das Entnehmen einer Mischung aus dem Medium und einer Kultur zur guten Homogenisierung und das Verdünnen um das 1- bis 10-Fache mit sterilem Wasser umfasst, um die zu testende Lösung des zu identifizierenden Stamms zu erhalten.

5. Verfahren nach Anspruch 3, wobei Schritt (4) Folgendes umfasst:
Schritt a: Herstellen einer Beschichtungslösung von 0,2-8,0 µg/mL aus dem Nanobody oder monoklonalen Antikörper von AFT-YJFZ01 unter Verwendung eines ELISA-Beschichtungspuffers, Zugeben der Beschichtungslösung in die ELISA-Platte, Entfernen der Beschichtungslösung aus der ELISA-Platte nach Stehenlassen über Nacht bei 4 °C oder bei 37 °C für nicht weniger als 2 h und Waschen der ELISA-Platte mit einer konventionellen Waschlösung für ELISA und anschließendes Durchführen einer Blockierung bei Raumtemperatur oder 37 °C für nicht weniger als 1 h unter Verwendung von Magermilchpulver mit einer Konzentration von nicht weniger als 1 % als Blockierungslösung, Verwerfen der Blockierungslösung und Waschen der ELISA-Platte mit der konventionellen Waschlösung für ELISA;
Schritt b: anschließendes angemessenes Verdünnen der zu testenden Lösung des zu identifizierenden Stamms mit einem konventionellen Phosphatpuffer mit einem nahezu neutralen pH-Wert, Zugeben der verdünnten zu testenden Lösung in Vertiefungen der ELISA-Platte, Durchführen einer Blockierung bei Raumtemperatur oder 37 °C für nicht weniger als 1 h, Verwerfen einer Flüssigkeit und Waschen der ELISA-Platte mit der konventionellen Waschlösung für ELISA;
Schritt c: anschließendes angemessenes Verdünnen des polyklonalen Antikörpers von AFT-YJFZ01 mit einem konventionellen Phosphatpuffer mit einem nahezu neutralen pH-Wert, Zugeben des verdünnten polyklonalen Antikörpers in Vertiefungen der ELISA-Platte, Durchführen einer Blockierung bei Raumtemperatur oder 37 °C für nicht weniger als 1 h, Verwerfen einer Flüssigkeit und Waschen der ELISA-Platte mit der konventionellen Waschlösung für ELISA;
Schritt d: anschließendes Verdünnen eines kommerziellen, mit Meerrettichperoxidase markierten Antikörpers mit einem konventionellen Phosphatpuffer mit einem nahezu neutralen pH-Wert nach Bedarf, Zugeben des verdünnten kommerziellen, mit Meerrettichperoxidase markierten Antikörpers in Vertiefungen der ELISA-Platte, Durchführen einer Blockierung bei Raumtemperatur oder 37 °C für nicht weniger als 1 h, Verwerfen einer Flüssigkeit und Waschen der ELISA-Platte mit der konventionellen Waschlösung für ELISA und
Schritt e: anschließendes sequenzielles Zugeben einer konventionellen Chromogenlösung für ELISA und der Stopplösung und schließlich Ablesen unter Verwendung des Mikroplatten-Lesegeräts und Berechnen eines Ergebniswerts des Gehalts an AFT-YJFZ01.

6. Verfahren zur Identifizierung, ob in einer Probe ein Aflatoxin produzierender Stamm mit hoher Toxigenität vorhanden ist, unter Verwendung des Verfahrens nach Anspruch 2, das insbesondere die folgenden Schritte umfasst:
(1) Bereitstellen eines Nanobodys oder monoklonalen Antikörpers des Indikatormoleküls AFT-YJFZ01 für die Toxigenität aflatoxigener Pilze;
(2) Bereitstellen eines polyklonalen Antikörpers des Indikatormoleküls AFT-YJFZ01 für die Toxigenität aflatoxigener Pilze;
(3) Herstellen einer zu testenden Lösung einer zu identifizierenden Probe: Kultivieren der zu identifizierenden Probe und Verdünnen, um die zu testende Lösung der zu identifizierenden Probe zu erhalten, und
(4) Identifizieren, ob in einer Probe ein Aflatoxin produzierender Stamm mit hoher Toxigenität vorhanden ist: Identifizieren der Probe unter Verwendung eines indirekten nicht-kompetitiven Doppelantikörper-Sandwich-Verfahrens, das die folgenden Schritte umfasst:
Beschichten einer ELISA-Platte mit dem Nanobody oder monoklonalen Antikörper von AFT-YJFZ01 und Waschen der Platte;
Durchführen einer Blockierung durch Zugeben einer Blockierungslösung und Waschen der Platte;
Zugeben der zu testenden Lösung der zu identifizierenden Probe, Ausführen einer Reaktion und Waschen der Platte;
Zugeben des polyklonalen Antikörpers von AFT-YJFZ01, Ausführen einer Reaktion und Waschen der Platte;
Zugeben eines mit Meerrettichperoxidase markierten Antikörpers, der an den polyklonalen Antikörper des Indikatormoleküls AFT-YJFZ01 bindet, Ausführen einer Reaktion und Waschen der Platte;
Zugeben einer Chromogenlösung und Ausführen einer Reaktion und
Zugeben einer Stopplösung, Durchführen einer Ablesung unter Verwendung eines Mikroplatten-Lesegeräts und Durchführen einer Berechnung, um einen Gehalt des Indikatormoleküls AFT-YJFZ01 für die Toxigenität aflatoxigener Pilze zu erhalten, und
(5) Auswerten der Identifizierungsergebnisse:
Je höher der Gehalt des Indikatormoleküls AFT-YJFZ01 für die Toxigenität aflatoxigener Pilze in der zu identifizierenden Probe ist, desto stärker ist die Aflatoxin-Produktionsfähigkeit, das heißt die Toxigenität, der zu identifizierenden Probe, wobei der Gehalt des Indikatormoleküls AFT-YJFZ01 für die Toxigenität aflatoxigener Pilze in der zu testenden Lösung der Probe pro Volumeneinheit basierend auf einem Ergebnis der Berechnung durch das Mikroplatten-Lesegerät erhalten wird und bestimmt wird, ob der Aflatoxin produzierende Stamm mit hoher Toxigenität in der zu identifizierenden Probe enthalten ist, wobei
das Indikatormolekül AFT-YJFZ01 für die Toxigenität aflatoxigener Pilze die in SEQ ID NO. 1 gezeigte Aminosäuresequenz aufweist.

7. Verfahren nach Anspruch 6, wobei Schritt (3) Folgendes umfasst: Einwiegen der zu identifizierenden Probe, Überführen der eingewogenen zu identifizierenden Probe in steriles Wasser, Durchführen eines Schüttelns bei Raumtemperatur, bis die erhaltene Lösung homogen ist, um eine homogene Dispersion der zu testenden Probe herzustellen, Entnehmen von 10-1000 µL der homogenen Dispersion der zu testenden Probe zur Zugabe in 6-600 mL eines konventionellen Czapek-Mediums oder anderer Medien, die für das Wachstum von toxinbildendem Aspergillus flavus geeignet sind, Durchführen einer Schüttelkultur bei 15-35 °C bei 200 ± 50 U/min nach 6-24 h und Entnehmen einer Probe, um die zu testende Lösung der zu identifizierenden Probe zu bilden, wobei die zu identifizierende Probe Erde, eine traditionelle chinesische Medizin, ein landwirtschaftliches Produkt oder ein Futtermittel ist.

8. Verfahren nach Anspruch 6, wobei Schritt (4) insbesondere Folgendes umfasst:
Schritt a: Herstellen einer Beschichtungslösung von 0,2-8,0 µg/mL aus dem Nanobody oder monoklonalen Antikörper von AFT-YJFZ01 unter Verwendung eines ELISA-Beschichtungspuffers, Zugeben der Beschichtungslösung in die ELISA-Platte, Entfernen der Beschichtungslösung aus der ELISA-Platte nach Stehenlassen über Nacht bei 4 °C oder bei 37 °C für nicht weniger als 2 h und Waschen der ELISA-Platte mit einer konventionellen Waschlösung für ELISA und anschließendes Durchführen einer Blockierung bei Raumtemperatur oder 37 °C für nicht weniger als 1 h unter Verwendung von Magermilchpulver mit einer Konzentration von nicht weniger als 1 % als Blockierungslösung, Verwerfen der Blockierungslösung und Waschen der ELISA-Platte mit der konventionellen Waschlösung für ELISA;
Schritt b: anschließendes angemessenes Verdünnen der zu testenden Lösung der zu identifizierenden Probe mit einem konventionellen Phosphatpuffer mit einem nahezu neutralen pH-Wert, Zugeben der verdünnten zu testenden Lösung in Vertiefungen der ELISA-Platte, Durchführen einer Blockierung bei Raumtemperatur oder 37 °C für nicht weniger als 1 h, Verwerfen einer Flüssigkeit und Waschen der ELISA-Platte mit der konventionellen Waschlösung für ELISA;
Schritt c: anschließendes angemessenes Verdünnen des polyklonalen Antikörpers von AFT-YJFZ01 mit einem konventionellen Phosphatpuffer mit einem nahezu neutralen pH-Wert, Zugeben des verdünnten polyklonalen Antikörpers in Vertiefungen der ELISA-Platte, Durchführen einer Blockierung bei Raumtemperatur oder 37 °C für nicht weniger als 1 h, Verwerfen einer Flüssigkeit und Waschen der ELISA-Platte mit der konventionellen Waschlösung für ELISA;
Schritt d: anschließendes Verdünnen eines kommerziellen, mit Meerrettichperoxidase markierten Antikörpers mit einem konventionellen Phosphatpuffer mit einem nahezu neutralen pH-Wert nach Bedarf, Zugeben des verdünnten kommerziellen, mit Meerrettichperoxidase markierten Antikörpers in Vertiefungen der ELISA-Platte, Durchführen einer Blockierung bei Raumtemperatur oder 37 °C für nicht weniger als 1 h, Verwerfen einer Flüssigkeit und Waschen der ELISA-Platte mit der konventionellen Waschlösung für ELISA und
Schritt e: anschließendes sequenzielles Zugeben einer konventionellen Chromogenlösung für ELISA und der Stopplösung und schließlich Ablesen unter Verwendung des Mikroplatten-Lesegeräts und Berechnen eines Ergebniswerts des Gehalts an AFT-YJFZ01.

9. Verfahren nach Anspruch 3 oder 6, wobei sich eine tierische Quelle des polyklonalen Antikörpers des Indikatormoleküls AFT-YJFZ01 für die Toxigenität aflatoxigener Pilze von einer tierischen Quelle des Nanobodys oder monoklonalen Antikörpers des Indikatormoleküls AFT-YJFZ01 für die Toxigenität aflatoxigener Pilze unterscheidet.

10. Verfahren nach Anspruch 3 oder 6, wobei Lösungen des Indikatormoleküls AFT-YJFZ01 für die Toxigenität aflatoxigener Pilze mit einer Reihe von Konzentrationsgradienten verwendet werden, um eine Eichkurve zu erstellen, und der Gehalt an AFT-YJFZ01 unter Verwendung der Eichkurve basierend auf den Ergebnissen des Mikroplatten-Lesegeräts erhalten wird.

## Revendications

1. Molécule indicatrice AFT-YJFZ01 pour la toxigénicité de champignons aflatoxigènes, présentant une séquence d'acides aminés représentée dans SEQ ID NO. 1.

2. Procédé d'identification de la toxigénicité de champignons aflatoxigènes dans un système, comprenant la détection d'une teneur en une molécule indicatrice AFT-YJFZ01 pour la toxigénicité de champignons aflatoxigènes, et l'identification de la toxigénicité de champignons aflatoxigènes dans un système sur la base de la teneur en la molécule indicatrice AFT-YJFZ01 pour la toxigénicité de champignons aflatoxigènes, la molécule indicatrice AFT-YJFZ01 pour la toxigénicité de champignons aflatoxigènes présentant une séquence d'acides aminés représentée dans SEQ ID NO. 1.

3. Procédé d'identification d'une capacité de production d'aflatoxine de souches productrices d'aflatoxine du genre *Aspergillus* à l'aide du procédé selon la revendication 2, comprenant :
(1) la fourniture d'un nanocorps ou d'un anticorps monoclonal de la molécule indicatrice AFT-YJFZ01 pour la toxigénicité de champignons aflatoxigènes ;
(2) la fourniture d'un anticorps polyclonal de la molécule indicatrice AFT-YJFZ01 pour la toxigénicité de champignons aflatoxigènes ;
(3) la préparation d'une solution à tester d'une souche à identifier : culture de la souche à identifier, et dilution pour obtenir la solution à tester de la souche à identifier ; et
(4) la détermination de la capacité de production d'aflatoxine de la souche à identifier :
l'identification de la capacité de production d'aflatoxine des souches productrices d'aflatoxine du genre *Aspergillus* en utilisant un procédé type sandwich d'anticorps double non-compétitif indirect, comprenant les étapes consistant à : revêtir une plaque ELISA avec le nanocorps ou l'anticorps monoclonal de l'AFT-YJFZ01, et laver la plaque ;
réaliser un blocage en ajoutant une solution de blocage, et laver la plaque ;
ajouter la solution à tester, réaliser une réaction, et laver la plaque ;
ajouter l'anticorps polyclonal de l'AFT-YJFZ01, réaliser une réaction, et laver la plaque ;
ajouter un anticorps marqué à la peroxydase de raifort qui se lie à l'anticorps polyclonal de la molécule indicatrice AFT-YJFZ01 pour la toxigénicité de champignons aflatoxigènes, réaliser une réaction, et laver la plaque ;
ajouter une solution chromogène, et réaliser une réaction ; et
ajouter une solution d'arrêt, réaliser une lecture en utilisant un lecteur de microplaques, et réaliser un calcul pour obtenir une teneur en la molécule indicatrice AFT-YJFZ01 pour la toxigénicité de champignons aflatoxigènes, dans lequel plus la teneur en la molécule indicatrice AFT-YJFZ01 pour la toxigénicité de champignons aflatoxigènes dans la solution à tester de la souche à identifier est élevée, plus la capacité de production d'aflatoxine de la souche productrice d'aflatoxine est forte ; et
la molécule indicatrice AFT-YJFZ01 pour la toxigénicité de champignons aflatoxigènes présente la séquence d'acides aminés représentée dans SEQ ID NO. 1.

4. Procédé selon la revendication 3, dans lequel l'étape (3) comprend la culture de la souche à identifier dans un milieu de Czapek conventionnel ou d'autres milieux adaptés pour la croissance de souches à une température ambiante de 15-35°C pendant au moins 12 heures, la prise d'un mélange du milieu et d'une culture à bien homogénéiser, et la réalisation d'une dilution de 1-10 fois avec de l'eau stérile pour obtenir la solution à tester de la souche à identifier.

5. Procédé selon la revendication 3, dans lequel l'étape (4) comprend :
Étape a : préparation d'une solution de revêtement de 0,2-8,0 µg/mL à partir du nanocorps ou de l'anticorps monoclonal de l'AFT-YJFZ01 en utilisant un tampon de revêtement ELISA, ajout de la solution de revêtement dans la plaque ELISA, élimination de la solution de revêtement de la plaque ELISA après une nuit au repos à 4°C ou à 37°C pendant au moins 2h, et lavage de la plaque ELISA avec une solution de lavage conventionnelle pour ELISA ; puis la réalisation d'un blocage à température ambiante ou à 37°C pendant au moins 1h en utilisant une poudre de lait écrémé présentant une concentration d'au moins 1% en tant que solution de blocage, l'élimination de la solution de blocage, et le lavage de la plaque ELISA avec la solution de lavage conventionnelle pour ELISA ;
Étape b : puis dilution de la solution à tester de la souche à identifier de manière appropriée avec un tampon phosphate conventionnel avec un pH proche du neutre, ajout de la solution à tester diluée dans des puits de la plaque ELISA, réalisation d'un blocage à température ambiante ou à 37°C pendant au moins 1h, élimination d'un liquide, et lavage de la plaque ELISA avec la solution de lavage conventionnelle pour ELISA ;
Étape c : puis dilution de l'anticorps polyclonal de l'AFT-YJFZ01 de manière appropriée avec un tampon phosphate conventionnel avec un pH proche du neutre, ajout de l'anticorps polyclonal dilué dans des puits de la plaque ELISA, réaliser un blocage à température ambiante ou 37°C pendant au moins 1h, élimination d'un liquide, et lavage de la plaque ELISA avec la solution de lavage conventionnelle pour ELISA ;
Étape d : puis dilution d'un anticorps marqué à la peroxydase de raifort commerciale avec un tampon phosphate conventionnel avec un pH proche du neutre selon les besoins, ajout de l'anticorps marqué à la peroxydase de raifort commerciale dilué dans des puits de la plaque ELISA, réalisation d'un blocage à température ambiante ou 37°C pendant au moins 1h, élimination d'un liquide, et lavage de la plaque ELISA avec la solution de lavage conventionnelle pour ELISA ; et
Étape e : puis ajout d'une solution chromogène conventionnelle pour ELISA et de la solution d'arrêt en séquence, et enfin lecture en utilisant le lecteur de microplaques, et calcul d'un résultat de teneur en AFT-YJFZ01.

6. Procédé d'identification de l'existence d'une souche productrice d'aflatoxine avec une toxigénicité élevée dans un échantillon à l'aide du procédé selon la revendication 2, comprenant spécifiquement les étapes suivantes :
(1) la fourniture d'un nanocorps ou d'un anticorps monoclonal de la molécule indicatrice AFT-YJFZ01 pour la toxigénicité de champignons aflatoxigènes ;
(2) la fourniture d'un anticorps polyclonal de la molécule indicatrice AFT-YJFZ01 pour la toxigénicité de champignons aflatoxigènes ;
(3) la préparation d'une solution à tester d'un échantillon à identifier : culture de l'échantillon à identifier, et dilution pour obtenir la solution à tester de l'échantillon à identifier ;
(4) l'identification de l'existence d'une souche productrice d'aflatoxine avec une toxigénicité élevée dans un échantillon : identification de l'échantillon en utilisant un procédé type sandwich d'anticorps double non-compétitif indirect, comprenant les étapes consistant à :
revêtir une plaque ELISA avec le nanocorps ou l'anticorps monoclonal de l'AFT-YJFZ01, et laver la plaque ;
réaliser un blocage en ajoutant une solution de blocage, et laver la plaque ;
ajouter la solution à tester de l'échantillon à identifier, réaliser une réaction, et laver la plaque ;
ajouter l'anticorps polyclonal de l'AFT-YJFZ01, réaliser une réaction, et laver la plaque ;
ajouter un anticorps marqué à la peroxydase de raifort qui se lie à l'anticorps polyclonal de la molécule indicatrice AFT-YJFZ01, réaliser une réaction, et laver la plaque ;
ajouter une solution chromogène, et réaliser une réaction ; et
ajouter une solution d'arrêt, réaliser une lecture en utilisant un lecteur de microplaques, et réaliser un calcul pour obtenir une teneur en la molécule indicatrice AFT-YJFZ01 pour la toxigénicité de champignons aflatoxigènes ; et
(5) l'évaluation des résultats d'identification :
plus la teneur en la molécule indicatrice AFT-YJFZ01 pour la toxigénicité de champignons aflatoxigènes dans l'échantillon à identifier est élevée, plus la capacité de production d'aflatoxine, c'est-à-dire la toxigénicité, de l'échantillon à identifier est forte, l'obtention de la teneur en la molécule indicatrice AFT-YJFZ01 pour la toxigénicité de champignons aflatoxigènes dans la solution à tester de l'échantillon par volume unitaire sur la base d'un résultat de calcul par le lecteur de microplaques, et la détermination de la présence de la souche productrice d'aflatoxine présentant une toxigénicité élevée dans l'échantillon à identifier ; dans lequel
la molécule indicatrice AFT-YJFZ01 pour la toxigénicité de champignons aflatoxigènes présente la séquence d'acides aminés représentée dans SEQ ID NO. 1.

7. Procédé selon la revendication 6, dans lequel l'étape (3) comprend : la pesée de l'échantillon à identifier, le transfert de l'échantillon à identifier pesé dans de l'eau stérile, la réalisation d'une agitation à température ambiante jusqu'à ce que la solution obtenue soit uniforme pour préparer une dispersion uniforme de l'échantillon à tester, la prise de 10-1000 µL de la dispersion uniforme de l'échantillon à ajouter dans 6-600 mL d'un milieu de Czapek conventionnel ou d'autres milieux adaptés à la croissance d'Aspergillus flavus producteur de toxines, la réalisation d'une culture par agitation à 15-35°C à 200 ± 50 tr/min après 6-24h, et la prise d'un échantillon pour former la solution à tester de l'échantillon à identifier ; dans lequel l'échantillon à identifier est de la terre, un médicament chinois traditionnel, un produit agricole ou un aliment.

8. Procédé selon la revendication 6, dans lequel l'étape (4) comprend spécifiquement :
Étape a : préparation d'une solution de revêtement de 0,2-8,0 µg/mL à partir du nanocorps ou de l'anticorps monoclonal de l'AFT-YJFZ01 en utilisant un tampon de revêtement ELISA, ajout de la solution de revêtement dans la plaque ELISA, élimination de la solution de revêtement de la plaque ELISA après une nuit au repos à 4°C ou à 37°C pendant au moins 2h, et lavage de la plaque ELISA avec une solution de lavage conventionnelle pour ELISA ; puis la réalisation d'un blocage à température ambiante ou à 37°C pendant au moins 1h en utilisant une poudre de lait écrémé présentant une concentration d'au moins 1% en tant que solution de blocage, l'élimination de la solution de blocage, et le lavage de la plaque ELISA avec la solution de lavage conventionnelle pour ELISA ;
Étape b : puis dilution de la solution à tester de l'échantillon à identifier de manière appropriée avec un tampon phosphate conventionnel avec un pH proche du neutre, ajout de la solution à tester diluée dans des puits de la plaque ELISA, réalisation d'un blocage à température ambiante ou 37°C pendant au moins 1h, élimination d'un liquide, et lavage de la plaque ELISA avec la solution de lavage conventionnelle pour ELISA ;
Étape c : puis dilution de l'anticorps polyclonal de l'AFT-YJFZ01 de manière appropriée avec un tampon phosphate conventionnel avec un pH proche du neutre, ajout de l'anticorps polyclonal dilué dans des puits de la plaque ELISA, réaliser un blocage à température ambiante ou 37°C pendant au moins 1h, élimination d'un liquide, et lavage de la plaque ELISA avec la solution de lavage conventionnelle pour ELISA ;
Étape d : puis dilution d'un anticorps marqué à la peroxydase de raifort commerciale avec un tampon phosphate conventionnel avec un pH proche du neutre selon les besoins, ajout de l'anticorps marqué à la peroxydase de raifort commerciale dilué dans des puits de la plaque ELISA, réalisation d'un blocage à température ambiante ou 37°C pendant au moins 1h, élimination d'un liquide, et lavage de la plaque ELISA avec la solution de lavage conventionnelle pour ELISA ; et
Étape e : puis ajout d'une solution chromogène conventionnelle pour ELISA et de la solution d'arrêt en séquence, et enfin lecture en utilisant le lecteur de microplaques, et calcul d'un résultat de teneur en AFT-YJFZ01.

9. Procédé selon la revendication 3 ou 6, dans lequel une source animale de l'anticorps polyclonal de la molécule indicatrice AFT-YJFZ01 pour la toxigénicité des champignons aflatoxigènes est différente d'une source animale du nanocorps ou de l'anticorps monoclonal de la molécule indicatrice AFT-YJFZ01 pour la toxigénicité des champignons aflatoxigènes.

10. Procédé selon la revendication 3 ou 6, dans lequel des solutions de la molécule indicatrice AFT-YJFZ01 pour la toxigénicité de champignons aflatoxigènes avec une série de gradients de concentration sont utilisées pour tracer une courbe standard, et la teneur en AFT-YJFZ01 est obtenue en utilisant la courbe standard sur la base des résultats du lecteur de microplaques.
